# EUROPEAN PATENT SPECIFICATION

(11) **EP 2 396 020 B1**
(45) Date of publication and mention of the grant of the patent: **06.03.2013**
(21) Application number: 10723909.7
(22) Date of filing: 10.02.2010
(51) Int. Cl.: A61K 38/10, C07K 7/08, C07K 14/435

(54) **NOVEL ANTIMICROBIAL PEPTIDES**
NEUE ANTIMIKROBIELLE PEPTIDE
NOUVEAUX PEPTIDES ANTIMICROBIENS

(30) Priority: 12.02.2009 CZ 20090083
(43) Date of publication of application: 21.12.2011
(73) Proprietor: Institute Of Organic Chemistry And Biochemistry As CR, V.V.I., 16610 Praha 6 (CZ)
(72) Inventor: MONINCOVA, Lenka, 747 06 Opava - Kylesovice (CZ); CEROVSKY, Vaclav, 252 63 Roztoky (CZ); SLANINOVA, Jirina, 147 00 Praha 4 (CZ); HOVORKA, Oldrich, 26301 Dobris (CZ); CVACKA Josef, 110 00 Praha 1 (CZ); VOBURKA, Zdenek, 186 00 Praha 8 (CZ); FUCIK, Vladimir, 170 00 Praha 7 (CZ); BOROVICKOVA, Lenka, 130 00 Praha 3 (CZ)
(74) Representative: Herman, Vaclav
(86) International application number: PCT/CZ2010/000016
(87) International publication number: WO 2010/091651

(56) References cited:
- WO-A2-01/10887
- WO-A2-96/28468
- WO-A2-2009/109156
- US-B2- 7 504 381
- MONINCOVA L ET AL: "NOVEL ANTIMICROBIAL PEPTIDES FROM THE VENOM OF EUSOCIAL BEE HALICTUS SEXCINCTUS (HYMENOPTERA: HALICTIDAE)" BIOPOLYMERS, NEW YORK, NY, US, vol. 92, no. 4, 12 June 2009 (2009-06-12), page 364, XP009138605 ISSN: 0006-3525
- NICOLAS P ET AL: "PEPTIDES AS WEAPONS AGAINST MICROORGANISMS IN THE CHEMICAL DEFENSE SYSTEM OF VERTEBRATES" ANNUAL REVIEW OF MICROBIOLOGY, ANNUAL REVIEWS, US LNKD- DOI:10.1146/ANNUREV.MI.49.100195.001425, vol. 49, 1 January 1995 (1995-01-01), pages 277-304, XP002911622 ISSN: 0066-4227
- MONINCOVÁ LENKA ET AL: "Novel antimicrobial peptides from the venom of the eusocial bee Halictus sexcinctus (Hymenoptera: Halictidae) and their analogs." AMINO ACIDS AUG 2010 LNKD- PUBMED:20198492, vol. 39, no. 3, August 2010 (2010-08), pages 763-775, XP002600587 ISSN: 1438-2199
- OTVOS L JR: "Antibacterial peptides isolated from insects." JOURNAL OF PEPTIDE SCIENCE : AN OFFICIAL PUBLICATION OF THE EUROPEAN PEPTIDE SOCIETY OCT 2000 LNKD- PUBMED:11071264, vol. 6, no. 10, October 2000 (2000-10), pages 497-511, XP002600588 ISSN: 1075-2617 cited in the application
- TOKE ORSOLYA: "Antimicrobial peptides: new candidates in the fight against bacterial infections." BIOPOLYMERS 2005 LNKD- PUBMED:15880793, vol. 80, no. 6, 2005, pages 717-735, XP002600589 ISSN: 0006-3525 cited in the application
- SLANINOVA J ET AL: "Toxicity study of antimicrobial peptides from wild bee venom and their analogs toward mammalian normal and cancer cells", PEPTIDES, ELSEVIER, AMSTERDAM, vol. 33, no. 1, 1 January 2012 (2012-01-01), pages 18-26, XP009160673, ISSN: 0196-9781, DOI: 10.1016/J.PEPTIDES.2011.11.002 [retrieved on 2011-11-07]
- SLANINOVA JIRINA ET AL: "The antifungal effect of peptides from hymenoptera venom and their analogs", CENTRAL EUROPEAN JOURNAL OF BIOLOGY, WARSAW : VERSITA, PL, vol. 6, no. 2, 1 April 2011 (2011-04-01), pages 150-159, XP009160674, ISSN: 1895-104X, DOI: 10.2478/S11535-010-0111-4
- LENKA MONINCOVÃ ET AL: "Novel antimicrobial peptides from the venom of the eusocial bee Halictus sexcinctus (Hymenoptera: Halictidae) and their analogs", AMINO ACIDS ; THE FORUM FOR AMINO ACID AND PROTEIN RESEARCH, SPRINGER-VERLAG, VI, vol. 39, no. 3, 3 March 2010 (2010-03-03), pages 763-775, XP019853816, ISSN: 1438-2199

## Description

### Field of the Invention

The invention involves two novel peptides isolated from natural sources of the formulas 1 and 2

H-Gly-Met Trp-Ser-Lys-Ile-Leu-Gly-His-Leu-Ile-Arg-NH₂ (1)

H-Gly-Lys- Trp-Met-Ser-Leu-Leu-Lys-His-Ile-Leu-Lys-NH₂ (2),

their new synthetic analogs ad their applications as antimicrobial, antifungal, antiparasitic and anticancer compounds.

### Background Art

Antimicrobial peptides are evolutionary conserved components of the host's innate immunity system that form the first line of defense against infections [1-3]. They have been identified in almost all classes of life. Among antimicrobial peptides, those from insects constitute a remarkable group [4]. Since insects are uniquely adapted to a variety of natural environments, often considered rather unhealthy by human standards, they have developed amazing resistance to bacterial infection, in which antimicrobial peptides play a major role. The significant advantage of antimicrobial peptides resides in their mechanism of action, which is markedly different from that of conventional antibiotics. Although the precise mechanism of the broad spectrum of antimicrobial activity of these peptides is not yet fully understood, they appear to act via a specific, but not receptor-mediated, formation of trans-membrane pores or ion channels on cellular membrane. This causes leakage of essential metabolites that results in the disruption of microbial cell structure and leads to cell death [5, 6]. In contrast to conventional antibiotics, they do not appear to induce microbial resistance and require only short time to induce killing. Quite peculiar groups of peptides with antimicrobial properties were identified in the venom of stinging insects such as hymenopterans [7]. The main function of hymenoptera venom is the self protection against predators or nest defense against intruders causing them pain and inflammation. Antimicrobial effect of peptides isolated from the venoms of wasps, bees, bumblebees and ants is rather secondary as in the case of peptides belonging to the group of mastoparans [8]. Nevertheless, antimicrobial effect of peptides isolated for example from the venom of wild bees is quite significant. As the growing resistance of bacterial pathogens to conventional antibiotics has become serious global health problem, this alarming situation resulted in a search for novel alternative to traditional antibiotics. Antimicrobial peptides appear to be among the most promising lead compounds for developing medicines in the fight against resistant pathogens. As regards of the synthetic availability of antimicrobial peptides isolated from the venom of wild bees, these peptides may found application in practical medicine.

### Description of the Invention

The invention involves novel peptides of the formulas (1) and (2) isolated from natural sources and their synthetic analogs

H-Gly-Met-Trp-Ser-Lys-Ile-Leu-Gly-His-Leu-Ile-Arg-NH₂ (1)

H-Gly-Lys-Trp-Met-Ser-Leu-Leu-Lys-His-Ile-Leu-Lys-NH₂ (2)

It is an aspect of the present invention that synthetic analogs of the peptides of formulas 1 and 2 have following formulas:

H-Gly-Met-Trp-Lys-Lys-Ile-Leu-Gly-His-Leu-Ile-Arg-NH₂

H-Gly-Met-Trp-Ser-Lys-Ile-Leu-Gly-His-Leu-Lys-Arg-NH₂

H-Gly-Met-Trp-Ser-Lys-Ile-Leu-Gly-His-Leu-Ile-Lys-NH₂

H-Gly-Lys-Trp-Lys-Ser-Leu-Leu-Lys-His-Ile-Leu-Lys-NH₂

It is an aspect of the present invention that peptides of general formulas 1 and 2 and their synthetic analogues, especially these mentioned above, can be used for the production of the medicament for the treatment of microbial, viral, parasitic and fungal diseases and for the treatment of cancer diseases.

It is a further aspect of the present invention that synthetic analogs of the peptides of formulas 1 and 2 have following formulas:

H-Gly-Lys-Trp-Lys-Lys-Ile-Leu-Gly-Lys-Leu-Ile-Arg-NH₂

H-Gly-Lys-Trp-Met-Lys-Leu-Leu-Lys-His-Ile-Leu-Lys-NH₂

H-Gly-Lys-Trp-Met-Ser-Leu-Leu-Lys-Lys-Ile-Leu-Lys-NH₂

It is an aspect of the present invention that synthetic peptides of formulas mentioned above can be used for the production of the medicament for the treatment of microbial, viral, parasitic and fungal diseases and for the treatment of cancer diseases.

### Detailed Description of the Invention

We isolated two peptides of the formula (1) and (2) from the venom reservoirs of primitively eusocial bees *Halictus sexcinctus* and named it halictin-I and halictin-II, respectively. The peptides 1, 2 and their synthetic anologs possess strong antimicrobial effect accompanied with moderate hemolytic activity.

Even if the primary role of halictines in the nature is not known, we deal with novel unique, so far not published, peptides with significant antimicrobial properties. These peptides possess antimicrobial properties thanks to the shape of their secondary structure which depends on their distinctive primary peptide sequence. Secondary structure of halictines is unordered in aqueous media, but it easily undergoes to the α-helical structure in the presence of α-helix inducing compounds such as trifluoroethanol or SDS or in an anisotropic environment of the bacterial cell membrane [9].

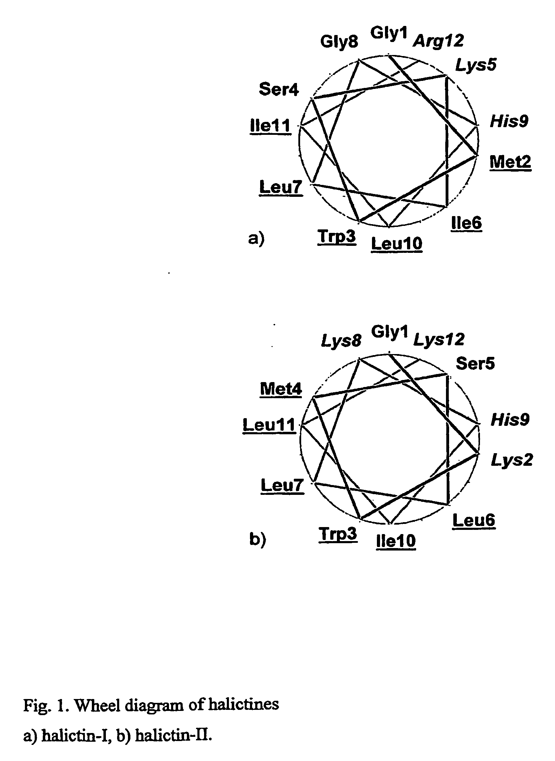

In the so called Edmundson wheel [10] projection (Fig.1), halictines have well-defined hydrophobic sectors with large aliphatic residues (red) on one side of the helix, and hydrophilic sector (black) dominated by cationic Lys, Arg and His residues (blue) on the opposite side of the helix. The ability of halictines to adopt such an amphipathic structure [11, 12] within bacterial cell membrane is one of the most important factors participating in the disruption of bacterial cell membrane finally resulting in the bacteria death. In addition, the cationic character [11] of halictines ensured by the presence of one lysine, one arginine and one histidine residue in halictin-I and by three lysine and one histidine in halictin-II located in hydrophilic sectors of the helices, plays also important role in the killing process. Due to this space arrangement, the positively charged faces of the peptides are attracted by electrostatic interaction to negatively charged phospholipids of bacterial cell membrane. The peptide accumulation on the membrane surface causes tension between the two leaflets of the lipidic membrane bilayer, which above a threshold peptide concentration leads to the disintegration and rupture of the bacterial cell membrane.

In anisotropic environment halictines may theoretically form the α-helical structures of three turns respectively. These helices are stabilized by hydrogen bonds in which every backbone NH group donates a hydrogen bond to the backbone C=O group of the preceding turn. The entire cationicity, hydrophobicity or amphipathicity of halictines can be modified by the replacement of the original amino acid residues in their sequences by other amino acids. It is known, that mild distortion of the helical structure may eliminate undesirable hemolytic activity of antimicrobial peptides, which is considered to be as a measure of peptide toxicity [9]. This may be achieved by replacing the amino acid residue in the central part of the peptide sequence by another amino acid residue with the property of helix breaker. These alterations in the sequences of halictines are done in an effort to increase their antimicrobial activity and reduce hemolytic activity [13]. Tables 1 and 2 show the advantageous examples of chemical modifications of peptides and their consequences on antimicrobial and hemolytic activity.

### Figures

Fig. 1 is a wheel diagram of halictines: halictin-I (a) and halictin-II (b).
Amino acids of hydrophobic sector are underlined. The amino acids (Gly, Ser) of hydrophilic sector on the opposite site of the helix a dominated by basic amino acid residues Lys, Arg and His (in italics).

### Examples

### Example 1

### Isolation from natural sources

Bee specimens of *Halictus sexcinctus* were collected in the vicinity of the village of Veska in Eastern Bohemia (Czech Republic) during June 2008 and were then kept frozen at -20°C. The venom reservoirs of six individuals were removed by dissection and their contents was extracted with 25 µl of a mixture of acetonitrile-water (1 : 1) containing 0.1% TFA (trifluoroacidic acid). The extract was centrifuged, and the supernatant was fractionated by RP-HPLC on the Vydac C-18, 250 × 4.6 mm; 5 µm, column at a 1 mL/min flow rate using a solvent gradient ranging from 5% to 70% acetonitrile/water/0.1% TFA over 60 min. The main peptide fractions detected by UV absorption at 220 nm were collected, the solvent was evaporated in a Speed-vac and the material was subjected to mass spectrometry and Edman degradation to detemnine its primary structure. Halictines represented the main peptide component of the venom extract.

### Example 2

### Synthesis of peptides of the general formula 1, 2 and their synthetic analogs.

These peptides may be synthesized according to following procedure: peptides were synthesized manually by using a solid-phase method in 5 mL polypropylene syringes with a bottom Teflon filter. Synthesis was done by using a Fmoc chemistry protocol on a Rink Amide MBHA resin (100 mg) with 0.7 mmol/g substitution. Protected amino acids were coupled in fourfold excess in dimethylformamide as solvent and N,N'-diisopropylcarbodiimide (7 equivalents)/1-hydroxybenzotriazole (5 equivalents) as coupling reagents. Crude peptides were deprotected and cleaved from the resin with a mixture of trifluoroacetic acid/1,2-ethanedithiol/water/thioanisol/triisopropylsilane (90 : 2.5 : 2.5 : 3 : 2) for 3.5 h and precipitated with *tert*-butyl methyl ether. Usually, crude peptides were obtained in the amount 70-80 mg. Part of this material (20 mg) of each peptide was purified by preparative RP-HPLC using a Vydac C-18 column (250 x 10 mm) at a flow rate 3.0 ml/min and using the gradient of solvents from 5% to 70% acetonitrile/water/0.1% TFA over 60 min yielding 8-11 mg of HPLC pure peptides. Their identities were verified by mass spectrometry as given in Table 1.

**Table 1**

| Amino acid sequences and MS data of halictines (1) and (2) and their analogs of general formula (1-A) and (2-A) | | | | |
|---|---|---|---|---|
| peptide | sequence | monoisotopic molecular mass (Da) | | |
| | | | calculated | found |
| (1) | H-Gly-Met-Trp-Ser-Lys-Ile-Leu-Gly-His-Leu-Ile-Arg-NH₂ | | 1408,81 | 1408;9 |
| (2) | H-Gly-Lys-Trp-Met-Ser-Leu-Leu-Lys-His-Ile-Leu-Lys-NH₂ | | 1451,88 | 1451,8 |
| (1-A/1) | H-Gly-Met-Trp-Lys-Lys-Ile-Leu-Gly-His-Leu-Ile-Arg-NH₂ | | 1449,88 | 1449,8 |
| (1-A/2) | H-Gly-Met-Trp-Ser-Lys-Ile-Leu-Gly-His-Leu-Lys-Arg-NH₂ | | 1423,82 | 1423,6 |
| (1-A/3) | H-Gly-Met-Trp-Ser-Lys-Ile-Leu-Gly-His-Leu-Ile-Lys-NH₂ | | 1380,81 | 1380,6 |
| (1-A/4) | H-Gly-Lys-Trp-Lys-Lys-Ile-Leu-Gly-Lys-Leu-Ile-Arg-NH₂ | | 1437,97 | 1438,0 |
| (2-A/1) | H-Gly-Lys-Trp-Lys-Ser-Leu-Leu-Lys-His-Ile-Leu-Lys-NH₂ | | 1448,93 | 1449,0 |
| (2-A/2) | H-Gly-Lys-Trp-Met-Lys-Leu-Leu-Lys-His-Ile-Leu-Lys-NH₂ | | 1492,94 | 1492,9 |
| (2-A/3) | H-Gly-Lys-Trp-Met-Ser-Leu-Leu-Lys-Lys-Ile-Leu-Lys-NH₂ | | 1442,92 | 1442,8 |

### Example 3

### Determination of antimicrobial activity

Antimicrobial activity was determined as a quantitative minimum inhibitory concentration (MIC) by observing bacterial growth in the presence of different concentrations of tested peptides. Mid-exponential phase bacteria were added to the solutions of the tested antimicrobial peptide at different concentrations (0.5 to 100 µM) in LB broth in multi-well plates. The plates were incubated at 37°C for 20 h while being continuously shaken in a Bioscreen C instrument (Finland). The absorbance was measured at 540 nm every 15 min and each peptide was tested at least 3 times in duplicate. In the experiments was routinely used 1.2 10³ - 7.5 10³ CFU of bacteria. Tetracycline in a concentration range of 0.1 - 50 µM was tested as a standard. The results of the tests are shown in Table 2.

**Table 2**

| Antimicrobial and hemolytic activity of halictines (1) and (2) and their analogs of general formula (1-A) and (2-A). | | | | | |
|---|---|---|---|---|---|
| peptide | | antimicrobial activity MIC [µM] | | | hemolytic activity LC₅₀ [µM] |
| | *B.s.* | *S.a.* | *E. c.* | *P.a.* | |
| (1) | 0,7 | 7,4 | 3,8 | 45 | 80 |
| (2) | 0,8 | 7,5 | 2,2 | 33 | 80 |
| (1-A/1) | 1,1 | 6,0 | 1,8 | 12,5 | 93 |
| (1-A/2) | 2,2 | 100 | 28 | 90 | >200 |
| (1-A/3) | 0,75 | 12,5 | 6.0 | 50 | 111 |
| (1-A/4) | 1,3 | 60,0 | 1,8 | 14,2 | >200 |
| (2-A/1) | 1,5 | 25 | 3 | 12,5 | 95 |
| (2-A/2) | 0,8 | 4,5 | 2,5 | 10,7 | 87,5 |
| (2-A/3) | 1,2 | 6,3 | 2,7 | 13,8 | 126 |

| | | | | | |
|---|---|---|---|---|---|
| *B.s,. Bacillus subtilis; S.a., Staphylococcus aureus; E.c., Escherichia coli; P.a.*, *Pseudomonnas aeruginosa;* | | | | | |

### Example 4

### Determination of hemolytic activity

The peptides in the concentration (1-200 µM) were incubated with rat red blood cells (5%) for 1 h at 37°C in a physiological solution at a final volume of 0.2 ml The samples were then centrifuged for 5 min at 250 g, and the absorbance of the supernatant was determined at 540 nm. Supernatants of red blood cells suspended in physiological solution and in 0.2% TRITON X100 in physiological solution served as controls for zero hemolysis (blank) and 100% hemolysis, respectively. The results of tests are shown in Table 2.

### References

1. Zasloff M. (2002) Antimicrobial peptides of multicellular organisms. Nature 415, 389-395
2. Hancock R.E.W., Sahl H.-G. Antimicrobial and host-defense peptides as new anti-infective therapeutic strategies. Natur. Bitech. 24, 1551-1557 (2006)
3. Toke O. Antimicrobial peptides: New candidates in the fight against bacterial infection. Biopolymers (Peptide Science) 80, 717-735 (2005)
4. Otvos L. Jr. Antimicrobial peptides isolated from insects. J. Peptide Sci. 6, 497-511 (2000)
5. Oren Z, Shai Y. Mode of action of linear amphipathic α-helical antimicrobial peptides. Biopolymers 47, 451-463 (1998)
6. Yeaman M.R., Yount N.Y. Mechanisms of antimicrobial peptide action and resistance. Pharm. Rev. 55,27-55 (2003)
7. Kuhn-Nentwig L. Antimicrobial and cytolytic peptides of venomous arthropods. Cell. Mol. Life Sci. 60, 2651-2668 (2003)
8. 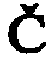 e 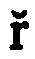 ovsk 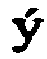 V., Slaninová J., Fu 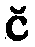 ík V, Hula 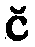 ová H, Boroviková L., Je 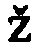 ek R and Bednárová L. New potent antimicrobial peptides from the venom of Polistinae wasps and their analogs. Peptides 29, 992-1003, (2008)
9. 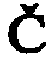 erovsk 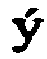 V., Hovorka O., Cva 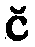 ka J., Voburka Z., Bednárová L., Borovi 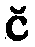 ková L., Slaninová J., and Fu 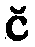 ík V. Melectin: a novel antimicrobial peptide from the venom of the cleptoparasitic bee Melecta albifrons. ChemBioChem 9, 2815-2821 (2008)
10. Schiffer M. and Edmundson A. B. Use of helical wheels to represent the structures of proteins and to identify segments with helical potential. Biophys. J. 7, 121-135 (1967)
11. Tossi A., Sandri L., Giangaspero A. Amphipathic, α-helical antimicrobial peptides. Biopolymers (Peptide Science) 55, 4-30 (2000)
12. Pathak N., Salas-Auvert R., Ruche G., Janna M.-H., McCarthy D., Harrison RG. Comparison of the effect of hydrophobicity, amphiphilicity, and α-helicity on the activities of antimicrobial peptides. Proteins: Struct Funct. Genet. 22; 182-86 (1995)
13. Conlon J.M., Al-Ghaferi N., Abraham B. and Leprince J. Strategies for transformation of naturally-occurring amphibian antimicrobial peptides into therapeutically valuable anti-infective agents. Methods 42, 349-357 (2007)

## Claims

1. Novel peptides of the formulas 1 and 2 isolated from natural sources
H-Gly-Met-Trp-Ser-Lys-Ile-Leu-Gly-His-Leu-Ile-Arg-NH₂ (1)
H-Gly-Lys-Trp-Met-Ser-Leu-Leu-Lys-His-Ile-Leu-Lys-NH₂ (2)

2. Synthetic analogs of the peptides of formulas 1 and 2 having following formulas:
H-Gly-Met-Trp-Lys-Lys-Ile-Leu-Gly-His-Leu-Ile-Arg-NH₂
H-Gly-Met-Trp-Ser-Lys-Ile-Leu-Gly-His-Leu-Lys-Arg-NH₂
H-Gly-Met-Trp-Ser-Lys-Ile-Leu-Gly-His-Leu-Ile-Lys-NH₂
H-Gly-Lys-Trp-Lys-Ser-Leu-Leu-Lys-His-Ile-Leu-Lys-NH₂

3. The use of peptides of general formulas 1 and 2 as well as their synthetic analogs according to claims 1 and 2 for the preparation of a medicament for the treatment of microbial, viral, parasitic and fungal diseases and for the treatment of cancer diseases.

4. Synthetic analogs of the peptides of formulas 1 and 2 having following formulas:
H-Gly-Lys-Trp-Lys-Lys-Ile-Leu-Gly-Lys-Leu-Ile-Arg-NH₂
H-Gly-Lys-Trp-Met-Lys-Leu-Leu-Lys-His-Ile-Leu-Lys-NH₂
H-Gly-Lys-Trp-Met-Ser-Leu-Leu-Lys-Lys-Ile-Leu-Lys-NH₂

5. The use of synthetic peptides according to claim 4 for the preparation of a medicament for the treatment of microbial, viral, parasitic and fungal diseases and for the treatment of cancer diseases.

## Patentansprüche

1. Neue antimikrobielle aus Naturquellen isolierte Peptide der Formeln 1 und 2
H-Gly-Met-Trp-Ser-Lys-Ile-Leu-Gly-His-Leu-Ile-Arg-NH₂ (1)
H-Gly-Lys-Trp-Met-Ser-Leu-Leu-Lys-His-Ile-Leu-Lys-NH₂ (2).

2. Synthetische Analoga der Peptide der Formeln 1 und 2 mit den folgenden Formeln:
H-Gly-Met-Trp-Lys-Lys-Ile-Leu-Gly-His-Leu-Ile-Arg-NH₂
H-Gly-Met-Trp-Ser-Lys-Ile-Leu-Gly-His-Leu-Lys-Arg-NH₂
H-Gly-Met-Trp-Ser-Lys-Ile-Leu-Gly-His-Leu-Ile-Lys-NH₂
H-Gly-Lys-Trp-Lys-Ser-Leu-Leu-Lys-His-Ile-Leu-Lys-NH₂

3. Verwendung der Peptide der allgemeinen Formeln 1 und 2 sowie deren synthetischen Analoga nach Ansprüchen 1 und 2 für die Herstellung eines Medikamentes zur Behnadlung von durch Mikroben, Viren, Parasiten und Pilzen verursachten Krankheiten und zur Behandlung von Krebskrankheiten.

4. Synthetische Analoga der Peptide der Formeln 1 und 2 mit den folgenden Formeln:
H-Gly-Lys-Trp-Lys-Lys-Ile-Leu-Gly-Lys-Leu-Ile-Arg-NH₂
H-Gly-Lys-Trp-Met-Lys-Leu-Leu-Lys-His-Ile-Leu-Lys-NH₂
H-Gly-Lys-Trp-Met-Ser-Leu-Leu-Lys-Lys-Ile-Leu-Lys-NH₂

5. Verwendung der synthetischen Peptide nach Anspruch 4 für die Herstellung eines Medikamentes zur Behandlung von durch Mikroben, Viren, Parasiten und Pilzen verursachten Krankheiten und zur Behandlung von Krebskrankheiten.

## Revendications

1. Nouveaux peptides de formules 1 et 2 isolés de sources naturelles
H-Gly-Met-Trp-Ser-Lys-Ile-Leu-Gly-His-Leu-Ile-Arg-NH₂ (1)
H-Gly-Lys-Trp-Met-Ser-Leu-Leu-Lys-His-Ile-Leu-Lys-NH₂ (2)

2. Analogues synthétiques de peptides de formules 1 and 2 ayant les formules suivante:
H-Gly-Met-Trp-Lys-Lys-Ile-Leu-Gly-His-Leu-Ile-Arg-NH₂
H-Gly-Met-Trp-Ser-Lys-Ile-Leu-Gly-His-Leu-Lys-Arg-NH₂
H-Gly-Met-Trp-Ser-Lys-Ile-Leu-Gly-His-Leu-Ile-Lys-NH₂
H-Gly-Lys-Trp-Lys-Ser-Leu-Leu-Lys-His-Ile-Leu-Lys-NH₂

3. L'usage des peptides de formules générales 1 et 2 ainsi que leurs analogues synthétiques selon les revendications 1 et 2 pour la préparation d'un médicament pour le traitement des maladies microbiennes, virales, parasitiques et fongiques et pour le traitment des maladies cancéreuses.

4. Analogues synthétiques de peptides de formules 1 and 2 ayant les formules suivante:
H-Gly-Lys-Trp-Lys-Lys-Ile-Leu-Gly-Lys-Leu-Ile-Arg-NH₂
H-Gly-Lys-Trp-Met-Lys-Leu-Leu-Lys-His-Ile-Leu-Lys-NH₂
H-Gly-Lys-Trp-Met-Ser-Leu-Leu-Lys-Lys-Ile-Leu-Lys-NH₂

5. L'usage des peptides synthétiques selon la revendication 4 pour la préparation d'un médicament pour le traitement des maladies microbiennes, virales, parasitiques et fongiques et pour le traitment des maladies cancéreuses.
